# EUROPEAN PATENT APPLICATION

(11) **EP 2 009 409 A2**
(43) Date of publication of application: **31.12.2008**
(21) Application number: 08159161.2
(22) Date of filing: 27.06.2008
(51) Int. Cl.: G01F 23/292, B27L 1/02

(54) **Method and apparatus for measuring the degree of fullness of a timber barking drum.**

(30) Priority: 27.06.2007 FI 20075490
(71) Applicant: Teknosavo Oy, 57100 Savonlinna (FI)
(72) Inventor: Hämäläinen, Hannu, 57710 Savonlinna (FI)
(74) Representative: LEITZINGER OY

(57) **Abstract**

The invention relates to a method and apparatus for measuring the degree of fullness of a pulpwood barking drum. The supply end of a drum (1) is illuminated with a light fixture (2), and a camera (3) is used for imaging through the drum. A dark area (4) visible in the image is interpreted as a pulpwood -loaded section of the drum, and an area (5) visible in illumination is interpreted as a void space. Thus obtained is true degree-of-fullness information regarding the drum, and a consistent degree of fullness can be maintained.

## Description

The invention relates to a method and apparatus for measuring the degree of fullness of a pulpwood barking drum.

The method and apparatus according to the invention lend themselves to monitoring the degree of fullness of a barking drum in wood-processing industry.

When a debarking line is controlled either by manual or automatic control, it is a fundamental requirement to have correct degree-of-fullness information of the drum. In the event that the degree-of-fullness measuring process does not proceed properly, a consistent degree of fullness of the drum cannot be maintained. In this case, the necessary control measures are based on false information, leading to either an over- or underload in the drum. A result of this is a less-than-optimal barking performance of the drum, which is manifested in a reduced capacity and also in increased losses of wood in the barking operation.

Traditionally, the measurement of the amount of pulpwood in a drum is performed by using either weighing sensors mounted on the drum's support structures or, in case the drum is hydrostatically supported, oil or water pressure data. A shortfall in these methods is that, as the measurement is calibrated with a specific type and amount of wood, a change in the type of wood brings forth a change also in weight information in relation to volume and furthermore a change in the drum's degree of fullness. For example, if the degree-of-fullness measurement has been performed with fresh birch wood and the drum is then supplied with dried-up long roundwood of birch, the same weight information requires an almost double amount of wood and hence the drum's true degree of fullness is significantly higher than that obtained with fresh wood.

It is an object of the invention to provide a method and apparatus apt for measuring the degree of fullness of a pulpwood barking drum, enabling the acquirement of true degree-of-fullness information for the barking drum, not affected by variations in the type of wood.

This object is achieved by a method presented in the appended claim 1 and by an apparatus presented in claim 4. Preferred embodiments of the invention are presented in the dependent claims.

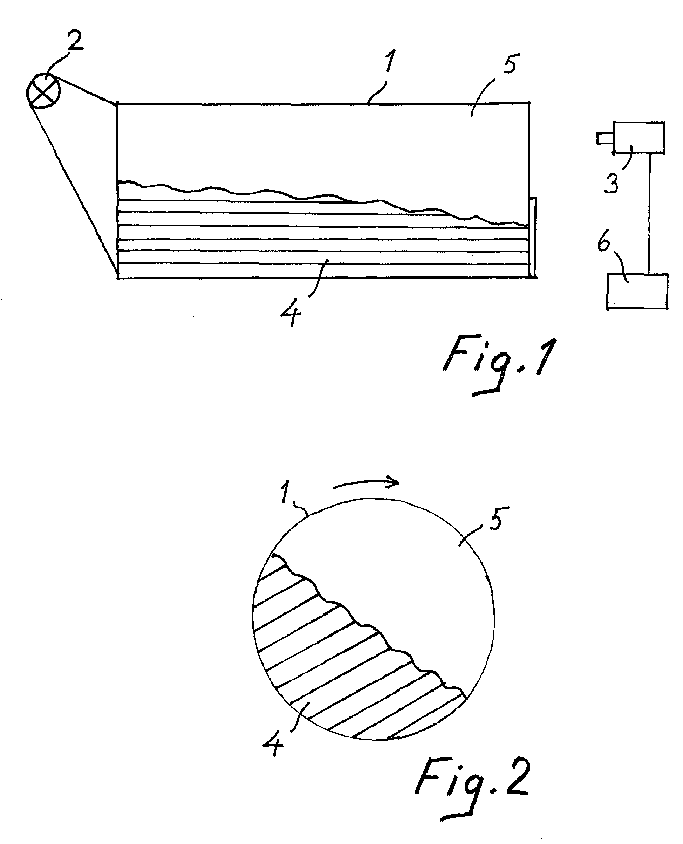

The invention will now be described more closely with reference to the accompanying drawing, in which
- Fig. 1: shows an apparatus implementing a method of the invention in a schematic lateral view (in a longitudinal section of the drum), and
- Fig. 2: shows a cross-sectional view of a barking drum 1, in which a method and apparatus of the invention are applied.

The method according to the invention is based on using a light fixture 2 for illuminating the inlet end of a drum, and on using a digital camera, preferably a matrix camera 3, for imaging through the drum. A dark area 4 visible in the camera image is a pulpwood -loaded section of the drum and an area 5 visible in illumination consists of void space. The proportions of a pulpwood area visible in a darker shade and an illuminated area provide a basis for calculating the drum's degree of fullness. Imaging is also feasible with a video camera by choosing the sweeping directions appropriately. The camera 3 can be a color camera, as well as a black-and-white camera. From an analog camera image can also be digitized a corresponding ratio of image fields. From a matrix camera image, however, the necessary information is obtained directly.

In the illustrated embodiment, the source of light 2 and the camera 3 are thus located at opposite ends of the drum 1, but can also be placed at the same end of the drum. Although, in a preferred embodiment of the invention, the source of light is positioned at the supply end of a drum and the camera at the discharge end, the invention is also functional with the camera at the supply end and the source of light at the discharge end, or, as pointed out earlier, with the camera and the source of light at the same end of a drum. The camera 3 is provided with image processing means 6 for determining said ratio of the image fields 4 and 5. The image processing means 6 may include e.g. a programmable processor (such as a microcomputer, a PC, etc.) for processing the image data and for calculating the relative proportions of illuminated and dark image fields.

The most important benefit offered by the invention is that obtained from a camera image is true degree-of-fullness information regarding the drum 1, not affected by variations in the type of wood. Thus, the drum's degree of fullness can be maintained optimal in terms of barking performance and losses of wood.

Otherwise the working of the barking drum 1 is based on a conventional method, wherein the drum is driven in rotation and logs are spinning in the drum and moving forward. The logs are debarked upon grinding against each other.

## Claims

1. A method for measuring the degree of fullness of a pulpwood barking drum, **characterized in that** a light fixture (2) is used for illuminating one end of a barking drum (1), a camera (3) is used for imaging through the barking drum, a dark area (4) visible in the image is interpreted as a pulpwood -loaded section of the drum and an area (5) visible in illumination as a void space.

2. A method according to claim 1, **characterized in that** illumination is applied to a supply end of the drum (1) and imaging through the drum is performed from a discharge end of the drum.

3. A method according to claim 1 or 2, **characterized in that** imaging is performed with a digital camera, preferably with the matrix camera (3).

4. An apparatus for measuring the degree of fullness of a pulpwood barking drum, **characterized in that** the apparatus includes a light fixture (2) for illuminating an end of a barking drum and a camera (3) located at the opposite end of a barking drum, which is directed for imaging through a barking drum (1), as well as image processing means (6) for determining the proportions of a dark area (4) visible in the image and an area (5) visible in illumination.

5. An apparatus according to claim 4, **characterized in that** the light fixture (2) is located at a supply end of the barking drum (1) and the camera (3) at a discharge end.

6. An apparatus according to claim 4 or 5, **characterized in that** the camera and the source of light are located at the same end of the drum.

7. An apparatus according to claim 4, 5 or 6, **characterized in that** the camera (3) is a digital camera, preferably a matrix camera.
